# EUROPEAN PATENT APPLICATION

(11) **EP 4 400 492 A1**
(43) Date of publication of application: **17.07.2024**
(21) Application number: 22864747.5
(22) Date of filing: 05.09.2022
(51) Int. Cl.: C07D 219/14, B01J 31/02, B01J 35/02, C07B 61/00, C07C 37/58, C07C 39/04

(54) **ACRIDINE COMPOUND**

(30) Priority: 06.09.2021 JP 2021144882
(71) Applicant: OSAKA UNIVERSITY, Suita-shi Osaka 565-0871 (JP); Otsuka Pharmaceutical Co., Ltd., Tokyo 101-8535 (JP)
(72) Inventor: OHKUBO, Kei, Suita-shi, Osaka 565-0871 (JP); AKAO, Yusuke, Osaka-shi, Osaka 540-0021 (JP); KOIZUMI, Yasuaki, Osaka-shi, Osaka 540-0021 (JP)
(74) Representative: Hoffmann Eitle
(86) International application number: PCT/JP2022/033207
(87) International publication number: WO 2023/033165

(57) **Abstract**

A compound represented by formula [I]: 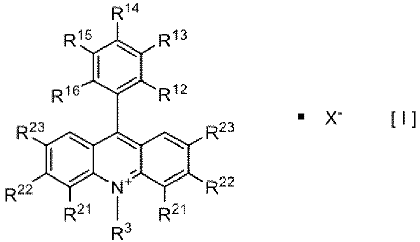 wherein R¹², R¹³, R¹⁴, R¹⁵, and R¹⁶ are the same or different and are each hydrogen, halogen, C₁₋₆ alkyl optionally substituted with halogen, or C₁₋₆ alkoxy optionally substituted with halogen; R²¹, R²², and R²³ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano; R³ is C₁₋₆ alkyl or the like; and X⁻ is an anion.

## Description

### Technical Field

The present invention relates to an acridine compound.

### Background Art

Phenols are important compounds widely used for phenol resins, various pharmaceutical products, and various chemical products such as dyes and disinfectants. The cumene method is generally known as an industrial synthesis method for phenol, which is a typical compound of the phenol family; however, its production process is complicated.

Cases using a photoredox catalyst with a quinolinium skeleton are disclosed as methods for producing phenol by direct oxidation of benzene by light irradiation (PTL 1, NPL 1, and NPL 2). These methods are not suitable as production methods since the production of phenol is about 50%.

As photoredox catalysts with an acridinium skeleton, for example, 9-phenyl-10-methylacridinium (Acr⁺-Ph) and 9-mesityl-10-methylacridinium (Acr⁺-Mes) are known (PTL 2 and PTL 3) .

### Citation List

### Patent Literature

PTL 1: JP2011-189224A
PTL 2: JP2005-145853A
PTL 3: WO2011/034071

### Non-patent Literature

NPL 1: Angew. Chem. Int. Ed., 50, 8652 (2011)
NPL 2: J. Am. Chem. Soc., 135, 5368 (2013)

### Summary of Invention

### Technical Problem

A quinolinium derivative represented by chemical formula (A): 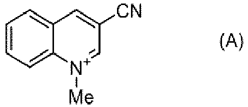 is a photoredox catalyst with high oxidizing power. The production of phenol from benzene using a photocatalyst requires high oxidizing power. However, the maximum absorption wavelength of the quinolinium derivative is about 310 nm, and the present inventors confirmed that the reaction stopped halfway because this wavelength was absorbed by phenol, which is the product.

The acridine compounds disclosed in PTL 2 and PTL 3 have oxidizing power as photoredox catalysts; however, their oxidizing power is insufficient.

An object of the present invention is to provide an acridine compound that has a maximum absorption wavelength at a wavelength other than the absorption wavelength of phenol, and that has extremely high oxidizing power as a photoredox catalyst.

### Solution to Problem

As a result of repeated studies to solve the above problem, the present inventors succeeded in the synthesis of a novel acridine compound represented by the following formula [I], and found that the compound has extremely high oxidizing power as a photoredox catalyst. The present invention has thus been completed.

Specifically, the present invention includes the following embodiments.

### [Item 1]

A compound represented by formula [I]: 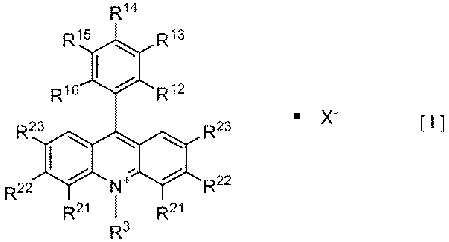 wherein
R¹², R¹³, R¹⁴, R¹⁵, and R¹⁶ are the same or different and are each hydrogen, halogen, C₁₋₆ alkyl optionally substituted with halogen, or C₁₋₆ alkoxy optionally substituted with halogen;
R²¹, R²², and R²³ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano;
R³ is C₁₋₆ alkyl or 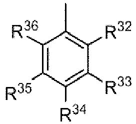 wherein R³², R³³, R³⁴, R³⁵, and R³⁶ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano; and
X⁻ is an anion;
provided that the following cases are excluded:
   1) all of R¹², R¹³, R¹⁴, R¹⁵, R¹⁶, R²¹, R²², and R²³ are hydrogen, and R³ is methyl or phenyl;
   2) all of R¹², R¹⁴, R¹⁶, and R³ are methyl, and R²³ is hydrogen or fluorine; and
   3) R¹² is hydrogen or methyl, R¹³ is hydrogen or methyl, R¹⁴ is hydrogen or methyl, R¹⁵ is hydrogen or methyl, R¹⁶ is hydrogen or methyl, R³ is methyl or unsubstituted phenyl, and R²³ is hydrogen.

### [Item 2]

The compound according to Item 1, wherein
R¹², R¹³, R¹⁴, R¹⁵, and R¹⁶ are the same or different and are each hydrogen, fluorine, chlorine, methyl, t-butyl, trifluoromethyl, or trifluoromethoxy;
R²¹, R²², and R²³ are the same or different and are each hydrogen, fluorine, chlorine, bromine, trifluoromethyl, methoxy, pentafluorosulfanyl, nitro, or cyano; and
R³ is methyl or 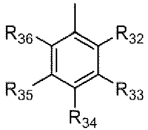 wherein R³², R³³, R³⁴, R³⁵, and R³⁶ are the same or different and are each hydrogen, fluorine, chlorine, bromine, methyl, trifluoromethyl, methoxy, pentafluorosulfanyl, nitro, or cyano.

### [Item 3]

The compound according to Item 1 or 2, wherein
R¹², R¹³, R¹⁴, R¹⁵, and R¹⁶ are the same or different and are each hydrogen, fluorine, methyl, t-butyl, trifluoromethyl, or trifluoromethoxy;
R²¹ and R²² are hydrogen, R²³ is fluorine; and
R³ is methyl or 4-fluorophenyl.

### [Item 4]

The compound according to any one of Items 1 to 3 selected from the following:

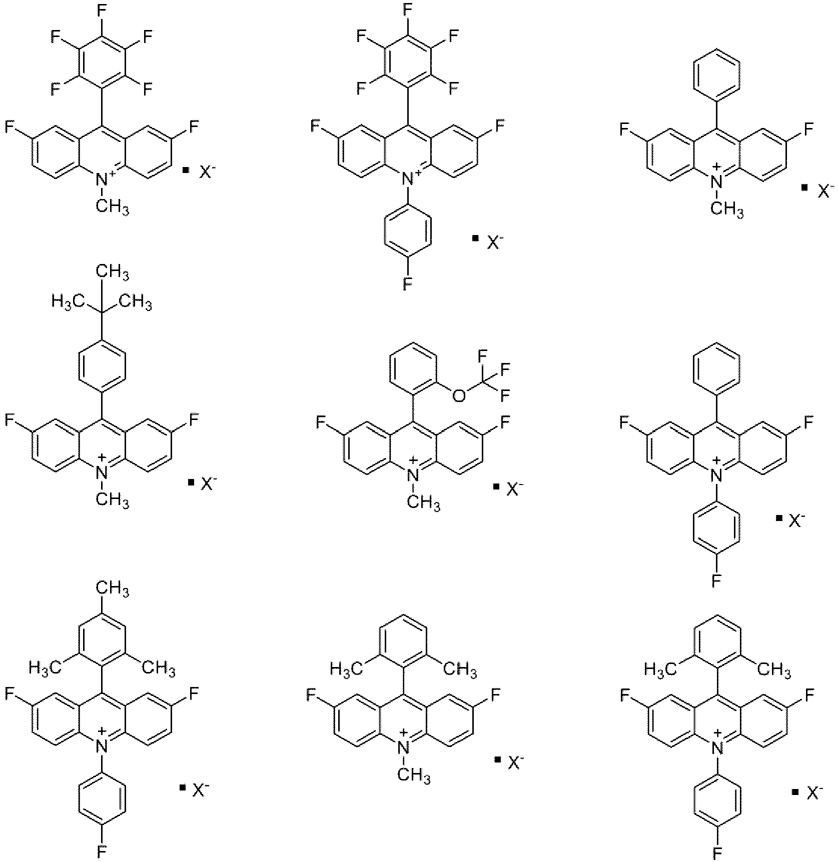

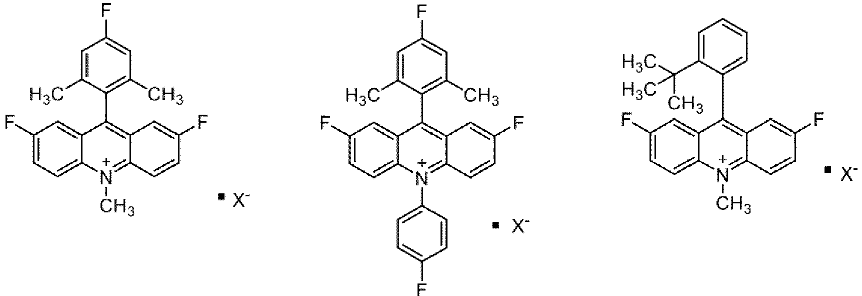

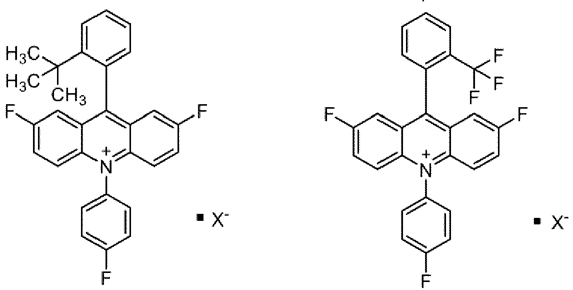

### [Item 5]

The compound according to any one of Items 1 to 4, wherein X⁻ is perchlorate ion (ClO₄⁻), hexafluorophosphate ion (PF₆⁻), or tetrafluoroborate ion (BF₄⁻).

### [Item 6]

A photoredox catalyst selected from the compound according to any one of Items 1 to 5.

### [Item 7]

Use of the compound according to any one of Items 1 to 5 as a photoredox catalyst.

### [Item 8]

A method for producing phenol from optionally substituted benzene, comprising irradiating optionally substituted benzene with visible light in the presence of the compound according to any one of Items 1 to 5.

### Advantageous Effects of Invention

The compound of the present invention has extremely high oxidizing power as a photoredox catalyst. In addition, the use of the compound of the present invention enables the efficient production of phenol by direct photooxidation of benzene.

### Brief Description of Drawings

Fig. 1 is a graph showing the correlation between reduction potential at the singlet excited state and electron affinity.
Fig. 2 shows graphs of the fluorescence lifetime.

### Description of Embodiments

The phrases and terms used in the present specification are described in detail below.

In the present specification, "halogen" is fluorine, chlorine, bromine, or iodine; preferably fluorine, chlorine, or bromine; and more preferably fluorine or chlorine.

In the present specification, examples of "C₁₋₆ alkyl" include C₁₋₆ linear or branched alkyl, and specific examples thereof include methyl, ethyl, n-propyl, isopropyl, n-butyl, isobutyl, sec-butyl, tert-butyl, n-pentyl, isopentyl, neopentyl, n-hexyl, isohexyl, 3-methylpentyl, and the like.

Further, "C₁₋₆ alkyl" also includes C₁₋₆ alkyl in which 1 to 7 hydrogen atoms are replaced by deuterium atoms.

In the present specification, examples of "C₁₋₆ alkyl optionally substituted with halogen" include C₁₋₆ linear or branched alkyl groups optionally substituted with 1 to 4 halogens. Specific examples thereof include methyl, ethyl, n-propyl, isopropyl, n-butyl, isobutyl, tert-butyl, sec-butyl, n-pentyl, isopentyl, neopentyl, n-hexyl, isohexyl, 3-methylpentyl, fluoromethyl, chloromethyl, bromomethyl, iodomethyl, difluoromethyl, dichloromethyl, dibromomethyl, trifluoromethyl, trichloromethyl, 2-fluoroethyl, 2-chloroethyl, 2,2,2-trifluoroethyl, 2,2,2-trichloroethyl, 1,1,2,2-tetrafluoroethyl, 3-chloropropyl, 2,3-dichloropropyl, 4,4,4-trichlorobutyl, 4-fluorobutyl, 5-chloropentyl, 3-chloro-2-methylpropyl, 5-bromohexyl, 5,6-dibromohexyl, and the like.

In the present specification, examples of "C₁₋₆ alkoxy optionally substituted with halogen" include C₁₋₆ linear or branched alkoxy groups optionally substituted with 1 to 4 halogens. Specific examples thereof include methoxy, ethoxy, n-propoxy, isopropoxy, n-butoxy, isobutoxy, tert-butoxy, sec-butoxy, n-pentoxy, isopentoxy, neopentoxy, n-hexyloxy, isohexyloxy, 3-methylpentyloxy, fluoromethoxy, chloromethoxy, bromomethoxy, iodomethoxy, difluoromethoxy, dichloromethoxy, dibromomethoxy, trifluoromethoxy, trichloromethoxy, 2-fluoroethoxy, 2-chloroethoxy, 2,2,2-trifluoroethoxy, 2,2,2-trichloroethoxy, 1,1,2,2-tetrafluoroethoxy, 3-chloropropoxy, 2,3-dichloropropoxy, 4,4,4-trichlorobutoxy, 4-fluorobutoxy, 5-chloropentyloxy, 3-chloro-2-methylpropoxy, 5-bromohexyloxy, 5,6-dibromohexyloxy, and the like.

In the present specification, examples of "sulfanyl optionally substituted with halogen" include pentafluorosulfanyl and the like.

In the present specification, "optionally substituted benzene" is benzene that may have 1 to 5 substituents. Examples of substituents include alkyl, halogen, an alkyl group having halogen as a substituent, and the like. Specific examples include benzene, toluene, fluorobenzene, chlorobenzene, bromobenzene, o-xylene, m-xylene, p-xylene, trifluoromethyl benzene, benzenesulfonic acid, and the like.

In the present specification, "Lewis acid" is an acid defined by G.N. Lewis in 1923. Specific examples include lithium tetrafluoroborate, aluminum chloride, yttrium(III) nitrate, silicon tetrachloride, ruthenium chloride, aluminum isopropoxide, aluminum(III) chloride, aluminum bromide, indium(III) chloride, copper(II) trifluoromethanesulfonate, lanthanum(III) trifluoromethanesulfonate, zinc(II) trifluoromethanesulfonate, silver trifluoromethanesulfonate, ytterbium(III) trifluoromethanesulfonate hydrate, scandium(III) trifluoromethanesulfonate, hafnium(IV) trifluoromethanesulfonate, cerium(III) trifluoromethanesulfonate, neodymium(III) trifluoromethanesulfonate, thulium(III) trifluoromethanesulfonate, yttrium(III) trifluoromethanesulfonate, tin(IV) chloride, tetraisopropyl orthotitanate, titanium(IV) chloride, boron trifluoride, and dicyclohexyl(trifluoromethanesulfonyloxy)borane. Preferred are lithium tetrafluoroborate and aluminum chloride, and more preferred is aluminum chloride.

In the present specification, X⁻ is not particularly limited as long as it is an anion. Examples include fluoride ion (F⁻), chloride ion (Cl⁻), bromide ion (Br⁻), iodide ion (I⁻), hydroxide ion (OH⁻), cyanide ion (CN⁻), nitrate ion (NO₃⁻), nitrite ion (NO₂⁻), hypochlorite ion (ClO⁻), chlorite ion (ClO₂⁻), chlorate ion (ClO₃⁻), perchlorate ion (ClO₄⁻), permanganate ion (MnO₄⁻), acetate ion (CH₃COO⁻), bicarbonate ion (HCO₃⁻), dihydrogen phosphate ion (H₂PO₄⁻), hydrogen sulfate ion (HSO₄⁻), hydrogen sulfide ion (HS⁻), thiocyanate ion (SCN-), hydrogen oxalate ion (H(COO)₂⁻), hexafluorophosphate ion (PF₆⁻), tetrafluoroborate ion (BF₄⁻), and the like. Preferred are perchlorate ion (ClO₄⁻), hexafluorophosphate ion (PF₆⁻), and tetrafluoroborate ion (BF-); and more preferred is perchlorate ion (ClO₄⁻).

In the present specification, the "base" is not particularly limited, but examples include inorganic bases, organic bases, and the like. Examples of inorganic bases include alkali metal hydroxides (e.g., lithium hydroxide, sodium hydroxide, and potassium hydroxide), alkaline earth metal hydroxides (e.g., magnesium hydroxide, calcium hydroxide, and barium hydroxide), alkali metal carbonates (e.g., sodium carbonate, potassium carbonate, and cesium carbonate), alkaline earth metal carbonates (e.g., magnesium carbonate, calcium carbonate, and barium carbonate), alkali metal hydrogen carbonates (e.g., sodium hydrogen carbonate and potassium hydrogen carbonate), alkali metal phosphates (e.g., sodium phosphate, potassium phosphate, and cesium phosphate), alkaline earth metal phosphates (e.g., magnesium phosphate and calcium phosphate), alkali metal alkoxides (e.g., sodium methoxide, sodium ethoxide, sodium tert-butoxide, and potassium tert-butoxide), alkali metal hydrides (e.g., sodium hydride and potassium hydride), sodium hydride, and the like. Examples of organic bases include trialkylamines (e.g., trimethylamine, triethylamine, and N,N-diisopropylethylamine (DIPEA)), dialkylamines (e.g., diethylamine and diisopropylamine), 4-dimethylaminopyridine (DMAP), N-methylmorpholine, picoline, 1,5-diazabicyclo[4.3.0]non-5-ene, 1,4-diazabicyclo[2.2.2]octane, 1,8-diazabicyclo[5.4.0]-7-undecene (DBU), and the like. One or more of these can be appropriately selected and used in combination.

In the present specification, "Bronsted base" is a base defined by Bronsted in 1923, and examples include inorganic bases and organic bases. Specific examples of inorganic bases include alkali metal hydrides (sodium hydride and potassium hydride) and alkaline earth metal hydrides (calcium hydride). Specific examples of organic bases include metal amides (lithium diisopropylamide, potassium hexamethyldisilazide, and lithium 2,2,6,6-tetramethylpiperidide).

In the present specification, the "palladium catalyst" is not particularly limited, but examples include tetravalent palladium catalysts, such as sodium hexachloropalladate(IV) tetrahydrate and potassium hexachloropalladate(IV); divalent palladium catalysts, such as [1,1'-bis(diphenylphosphino)ferrocene]palladium(II) dichloride dichloromethane adduct (Pd(dppf)Cl₂·CH₂Cl₂), (2-dicyclohexylphosphino-2',4',6'-triisopropyl-1,1'-biphenyl)[2-(2'-amino-1,1'-biphenyl)]palladium(II) methanesulfonate (XPhos Pd G3), [(2-dicyclohexylphosphino-3,6-dimethoxy-2',4',6'-triisopropyl-1,1'-biphenyl)-2-(2'-amino-1,1'-biphenyl)]palladium(II) methanesulfonate (BrettPhos Pd G3), palladium(II) chloride, palladium(II) bromide, palladium(II) acetate, palladium(II) acetylacetonate, dichlorobis(benzonitrile)palladium (II), dichlorobis(acetonitrile)palladium(II), dichlorobis(triphenylphosphine)palladium(II), dichlorotetraammine palladium(II), dichloro(cycloocta-1,5-diene)palladium(II), palladium(II) trifluoroacetate, and 1,1'-bis(diphenylphosphino)ferrocene dichloropalladium(II)-dichloromethane complex; zero-valent palladium catalysts, such as tris(dibenzylideneacetone)dipalladium(0) (Pd₂(dba)₃), tris(dibenzylideneacetone)dipalladium(0) chloroform complex, and tetrakis(triphenylphosphine)palladium(0) (Pd(PPh₃)₄); and the like. These palladium catalysts may be used singly or in combination of two or more.

In the present specification, specific examples of the "leaving group" include halogen, C₁₋₁₈ alkanesulfonyl, lower alkanesulfonyloxy, arylsulfonyloxy, aralkylsulfonyloxy, perhaloalkanesulfonyloxy, sulfonio, toluenesulfoxy, and the like. Halogen is preferred as the leaving group in the present reaction.

The "halogen" is fluorine, chlorine, bromine, or iodine.

Examples of the "C₁₋₁₈ alkanesulfonyl" include C₁₋₁₈ linear or branched alkanesulfonyl, and specific examples thereof include methanesulfonyl, 1-propanesulfonyl, 2-propanesulfonyl, 1-butanesulfonyl, cyclohexanesulfonyl, 1-dodecanesulfonyl, 1-octadecanesulfonyl, and the like.

Examples of the "lower alkanesulfonyloxy" include C₁₋₆ linear or branched alkanesulfonyloxy, and specific examples thereof include methanesulfonyloxy, ethanesulfonyloxy, 1-propanesulfonyloxy, 2-propanesulfonyloxy, 1-butanesulfonyloxy, 3-butanesulfonyloxy, 1-pentanesulfonyloxy, 1-hexanesulfonyloxy, and the like.

Examples of the "arylsulfonyloxy" include phenylsulfonyloxy optionally having, on the phenyl ring, 1 to 3 groups selected from the group consisting of C₁₋₆ linear or branched alkyl, C₁₋₆ linear or branched alkoxy, nitro, and halogen as substituents, naphthylsulfonyloxy, and the like. Specific examples of the "phenylsulfonyloxy optionally having ... substituents" include phenylsulfonyloxy, 4-methylphenylsulfonyloxy, 2-methylphenylsulfonyloxy, 4-nitrophenylsulfonyloxy, 4-methoxyphenylsulfonyloxy, 2-nitrophenylsulfonyloxy, 3-chlorophenylsulfonyloxy, and the like.

Specific examples of the "naphthylsulfonyloxy" include α-naphthylsulfonyloxy, β-naphthylsulfonyloxy, and the like.

Examples of the "aralkylsulfonyloxy" include phenyl-substituted C₁₋₆ linear or branched alkanesulfonyloxy optionally having, on the phenyl ring, 1 to 3 groups selected from the group consisting of C₁₋₆ linear or branched alkyl, C₁₋₆ linear or branched alkoxy, nitro, and halogen as substituents; naphthyl-substituted C₁₋₆ linear or branched alkanesulfonyloxy optionally having, on the phenyl ring, 1 to 3 groups selected from the group consisting of C₁₋₆ linear or branched alkyl, C₁₋₆ linear or branched alkoxy, nitro, and halogen as substituents; and the like. Specific examples of the "phenyl-substituted alkanesulfonyloxy" include benzylsulfonyloxy, 2-phenylethylsulfonyloxy, 4-phenylbutylsulfonyloxy, 4-methylbenzylsulfonyloxy, 2-methylbenzylsulfonyloxy, 4-nitrobenzylsulfonyloxy, 4-methoxybenzylsulfonyloxy, 3-chlorobenzylsulfonyloxy, and the like. Specific examples of the "naphthyl-substituted alkanesulfonyloxy" include α-naphthylmethylsulfonyloxy, β-naphthylmethylsulfonyloxy, and the like.

Specific examples of the "perhaloalkanesulfonyloxy" include trifluoromethanesulfonyloxy and the like.

Specific examples of the "sulfonio" include dimethylsulfonio, diethylsulfonio, dipropylsulfonio, di(2-cyanoethyl)sulfonio, di(2-nitroethyl)sulfonio, di-(aminoethyl)sulfonio, di(2-methylaminoethyl)sulfonio, di-(2-dimethylaminoethyl)sulfonio, di-(2-hydroxyethyl)sulfonio, di-(3-hydroxypropyl)sulfonio, di-(2-methoxyethyl)sulfonio, di-(2-carbamoylethyl)sulfonio, di-(2-carbamoylethyl)sulfonio, di-(2-carboxyethyl)sulfonio, di-(2-methoxycarbonylethyl)sulfonio, diphenylsulfonio, and the like.

In the present specification, the "solvent" may be a solvent inert to the reaction. Examples include water, ethers (e.g., dioxane, tetrahydrofuran, diethyl ether, 1,2-dimethoxyethane, diethylene glycol dimethyl ether, and ethylene glycol dimethyl ether), halogenated hydrocarbons (e.g., methylene chloride, chloroform, 1,2-dichloroethane, and carbon tetrachloride), aromatic hydrocarbons (e.g., benzene, toluene, xylene, and chlorobenzene), C₁₋₄ alcohols (e.g., methanol, ethanol, and isopropanol), and polar solvents (e.g., N,N-dimethylformamide (DMF), N-methylpyrrolidone (NMP), dimethyl sulfoxide (DMSO), hexamethylphosphoric triamide, and acetonitrile). These solvents may be used singly or in combination of two or more.

The substituents in the compound represented by formula [I] of the present invention (hereinafter referred to as "compound [I]") are each described below.

In compound [I], R¹², R¹³, R¹⁴, R¹⁵, and R¹⁶ are the same or different and are each hydrogen, halogen, C₁₋₆ alkyl optionally substituted with halogen, or C₁₋₆ alkoxy optionally substituted with halogen; preferably hydrogen, fluorine, chlorine, methyl, t-butyl, trifluoromethyl, or trifluoromethoxy; and more preferably hydrogen, fluorine, methyl, t-butyl, trifluoromethyl, or trifluoromethoxy.

In compound [I], R²¹, R²², and R²³ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano; preferably hydrogen, fluorine, chlorine, bromine, trifluoromethyl, methoxy, pentafluorosulfanyl, nitro, or cyano; and preferably hydrogen or fluorine.

In compound [I], R³ is C₁₋₆ alkyl, and preferably methyl.

In another embodiment, in compound [I], R³ is 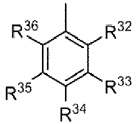 wherein R³², R³³, R³⁴, R³⁵, and R³⁶ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano; preferably hydrogen, fluorine, chlorine, bromine, methyl, trifluoromethyl, methoxy, pentafluorosulfanyl, nitro, or cyano; more preferably 4-fluorophenyl, 2,4-difluorophenyl, 2,4,6-trifluorophenyl, 2,3,4,5,6-pentafluorophenyl, 4-fluoro-2-methylphenyl, 4-fluoro-2,6-dimethylphenyl, 4-trifluoromethylphenyl, 4-cyanophenyl, 4-nitrophenyl, or 4-pentafluorosulfanylphenyl.

However, the following cases are excluded: all of R¹², R¹³, R¹⁴, R¹⁵, R¹⁶, R²¹, R²², and R²³ are hydrogen, and R³ is methyl or phenyl; all of R¹², R¹⁴, R¹⁶, and R³ are methyl, and R²³ is hydrogen or fluorine; R¹² is hydrogen or methyl, R¹³ is hydrogen or methyl, R¹⁴ is hydrogen or methyl, R¹⁵ is hydrogen or methyl, R¹⁶ is hydrogen or methyl, R³ is methyl or unsubstituted phenyl, and R²³ is hydrogen; and all of R¹², R¹⁴, R¹⁶, and R³ are methyl at the same time.

In another embodiment, in compound [I], R²¹ and R²² are hydrogen, and R²³ is halogen, C₁₋₆ alkyl optionally substituted with halogen, sulfanyl optionally substituted with halogen, nitro, or cyano, and preferably fluorine, chlorine, bromine, trifluoromethyl, pentafluorosulfanyl, nitro, or cyano.

In another embodiment, in compound [I], R²² and R²³ are hydrogen, and R²¹ is halogen, C₁₋₆ alkyl optionally substituted with halogen, sulfanyl optionally substituted with halogen, nitro, or cyano, and preferably fluorine, chlorine, trifluoromethyl, pentafluorosulfanyl, nitro, or cyano.

In another embodiment, in compound [I], R²¹ and R²³ are hydrogen, and R²² is halogen, C₁₋₆ alkyl optionally substituted with halogen, sulfanyl optionally substituted with halogen, nitro, or cyano, and preferably fluorine, chlorine, trifluoromethyl, pentafluorosulfanyl, nitro, or cyano.

In still another embodiment, in compound [I], R²² is hydrogen, and R²¹ and R²³ are fluorine.

In another embodiment, in compound [I], R²³ and R³⁴ are each hydrogen, halogen, alkyl optionally substituted with halogen, sulfanyl optionally substituted with halogen, cyano, or nitro, and preferably hydrogen, fluorine, trifluoromethyl, pentafluorosulfanyl, cyano, or nitro.

Preferred specific embodiments are the following compounds.

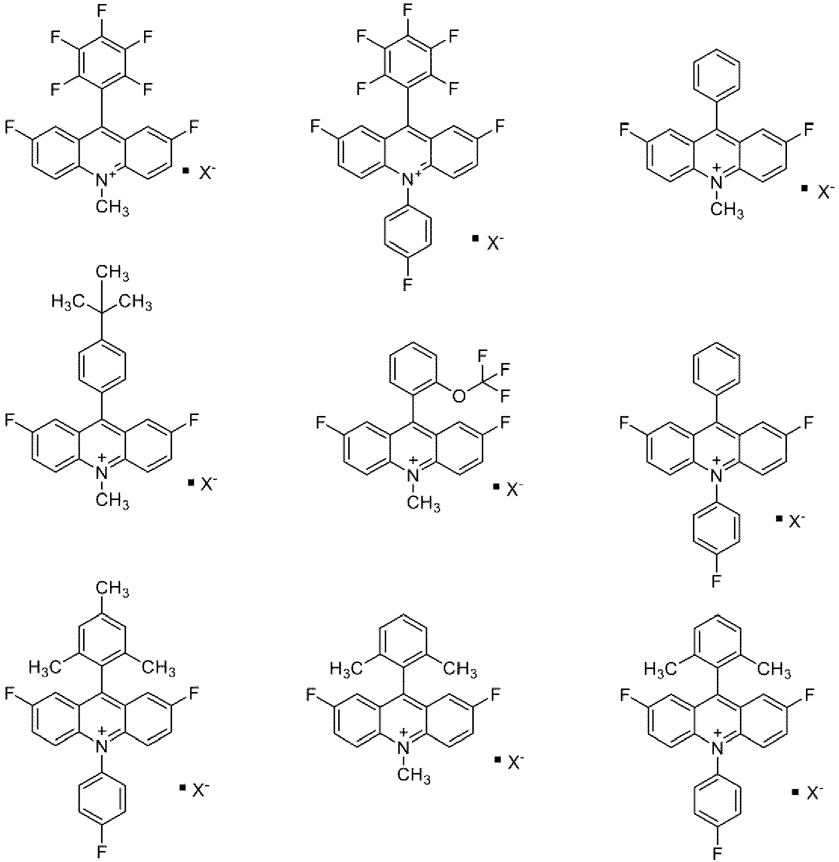

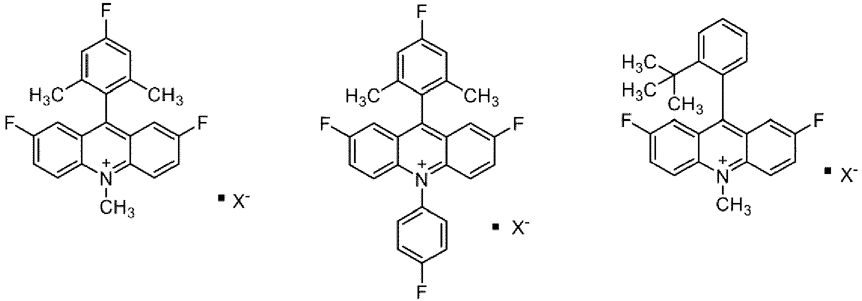

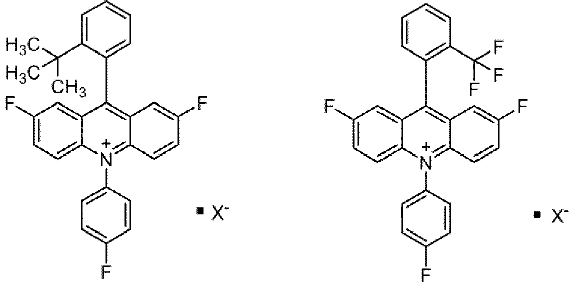

In a preferred specific embodiment, the anion (X⁻) that forms a salt with an acridine compound is perchlorate ion (ClO₄⁻), hexafluorophosphate ion (PF₆⁻), or tetrafluoroborate ion (BF₄⁻).

In a preferred specific embodiment, the anion (X⁻) that forms a salt with an acridine compound is perchlorate ion (ClO₄⁻).

In the present specification, presentation of preferred embodiments and options regarding different features of the compound, method, and composition of the present invention also includes presentation of combinations of preferred embodiments and options regarding the different features, as long as these are combinable and consistent.

The method for producing compound [I] is described below. Compound [I] can be produced, for example, by any of the production methods shown below. The production methods shown below are merely examples, and the method for producing compound [I] is not limited thereto.

In the following reaction formulas, when performing an alkylation reaction, a hydrolysis reaction, an amination reaction, an esterification reaction, an amidation reaction, an etherification reaction, a nucleophilic substitution reaction, an addition reaction, an oxidation reaction, a reduction reaction, etc., these reactions can be performed according to known methods. Examples of such methods include those described in, for example, Experimental Chemistry Course (5th ed., edited by the Chemical Society of Japan, Maruzen Co., Ltd.); Organic Functional Group Preparations, 2nd ed., Academic Press, Inc., published in 1989; Comprehensive Organic Transformations, VCH Publishers Inc., published in 1989; and P.G.M. Wuts and T.W. Greene, "Greene's Protective Groups in Organic Synthesis" (4th ed., 2006).

### General synthetic route for compound [I]

### 1) Method (1) for producing compound [IV]

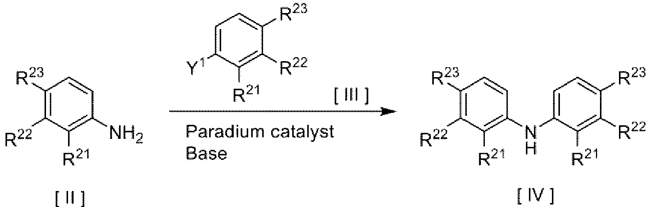

wherein Y¹ is a leaving group, and the other symbols are as defined above.

Compound [IV], which is an intermediate of compound [I] of the present invention, can be produced by the reaction shown in the above reaction formula. Specifically, compound [II] and compound [III] are subjected to a cross-coupling reaction in the presence of a base using a palladium catalyst in a solvent inert to the reaction, whereby compound [IV] can be produced.

Compound [II] and compound [III] are both known compounds, or compounds that can be easily produced by known methods.

Other reaction conditions (reaction temperature, reaction time, etc.) can be appropriately determined based on known cross-coupling reactions.

### 2) Method (1) for producing compound [VI]

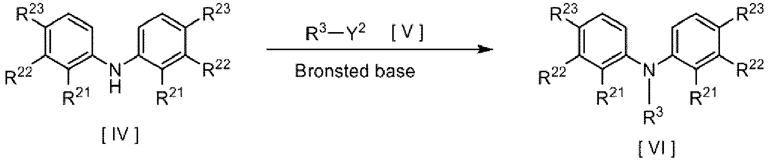

wherein Y² is a leaving group, and the other symbols are as defined above.

Compound [VI], which is an intermediate of compound [I] of the present invention, can be produced by the reaction shown in the above reaction formula. Specifically, compound [IV] and compound [V] are reacted in the presence of a Bronsted base in a solvent inert to the reaction, whereby compound [VI] can be produced.

Compound [V] is a known compound, or a compound that can be easily produced by a known method.

Other reaction conditions (reaction temperature, reaction time, etc.) can be appropriately determined based on known reactions.

### 3) Method (2) for producing compound [VI]

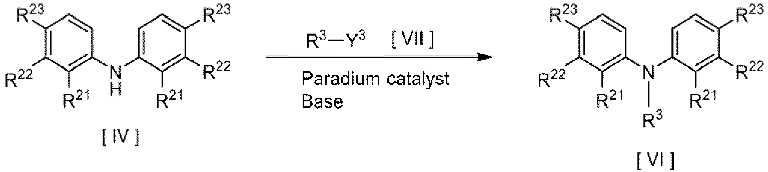

wherein Y³ is a leaving group, and the other symbols are as defined above.

Compound [VI], which is an intermediate of compound [I] of the present invention, can be produced by the reaction shown in the above reaction formula. Specifically, compound [IV] and compound [VII] are reacted in the presence of a base using a palladium catalyst in a solvent inert to the reaction, whereby compound [VI] can be produced.

Compound [VII] is a known compound, or a compound that can be easily produced by a known method.

Other reaction conditions (reaction temperature, reaction time, etc.) can be appropriately determined based on known reactions.

### 4) Method (1) for producing compound [I]

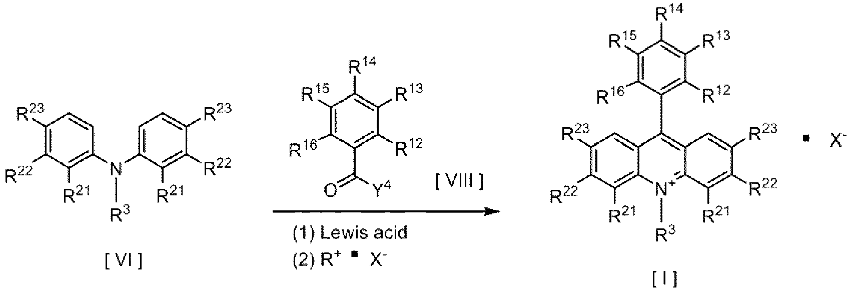

wherein Y⁴ is a leaving group, R⁺ is a cation, X⁻ is an anion, and the other symbols are as defined above.

Compound [I] of the present invention can be produced by the reaction shown in the above reaction formula. Specifically, compound [VI] and compound [VIII] are reacted in the presence of a Lewis acid without a solvent or in a solvent inert to the reaction. This step may be performed under microwave irradiation. Further, a salt (R⁺·X⁻) is acted, whereby compound [I] can be produced. Here, R is, for example, an alkali metal atom, and preferably sodium.

Compound [VIII] is a known compound, or a compound that can be easily produced by a known method.

Other reaction conditions (reaction temperature, reaction time, etc.) can be appropriately determined based on known reactions.

### 5) Method (2) for producing compound [I]

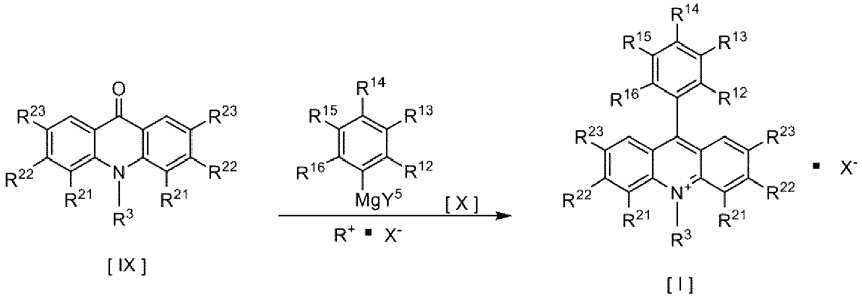

wherein Y⁵ is a leaving group, and the other symbols are as defined above.

Compound [I] of the present invention can be produced by the reaction shown in the above reaction formula. Specifically, compound [IX] and compound [X] are reacted in a solvent inert to the reaction, and a salt (R⁺·X⁻) is further acted, whereby compound [I] can be produced.

Compound [IX] and compound [X] are both known compounds, or compounds that can be easily produced by known methods.

Compound [X] can also be produced from its precursor halide and magnesium, and can be used as it is in the present reaction.

Other reaction conditions (reaction temperature, reaction time, etc.) can be appropriately determined based on known reactions.

In each of the reactions of the above reaction formulas, the product can be used as the reaction liquid or as the crude product for the next reaction. Alternatively, it can be isolated from the reaction mixture according to a conventional method and easily purified by a general separation method. Examples of general separation methods include recrystallization, distillation, and chromatography.

The starting raw material compound, intermediate compound, and target compound, as well as compound [I] in each of the above steps include geometric isomers, stereoisomers, optical isomers, and tautomers. Various isomers can be separated by common optical resolution methods. Such optical isomers can also be produced from suitable optically active raw material compounds.

Compound [I] can be produced by the synthesis method shown in each of the above reaction formulas or by a method equivalent thereto.

The raw material compounds in the production of compound [I] may be commercially available or produced according to known methods or equivalent methods, unless a specific production method is described.

The starting raw material compound and target compound in each of the above steps can be used in appropriate salt forms. Such salts include those similar to those exemplified below as salts of compound [I].

The present invention also includes various hydrates, solvates, and crystal polymorphs of compound [I].

Compound [I] includes compounds in which one or more atoms are replaced by one or more isotopic atoms. Examples of isotopic atoms include deuterium (²H), tritium (³H), ¹³C, ¹⁵N, ¹⁸O, and the like.

Compound [I] may be a co-crystal or a co-crystal salt. Co-crystals or co-crystal salts refer to crystalline substances composed of two or more unique solids at room temperature, each having different physical properties (e.g., structure, melting point, and heat of fusion). Co-crystals and co-crystal salts can be produced by applying known co-crystallization methods.

Since the excitation wavelength (maximum absorption wavelength) of compound [I] is visible light (360 nm to 830 nm), and preferably 365 nm to 435 nm, it can be used as a photoredox catalyst to oxidize substances (various compounds).

Since compound [I] has strong oxidizing power, it can be used as a photoredox catalyst to oxidize an aromatic compound, thereby converting the aromatic hydrogen into a hydroxyl group in high yield. For example, compound [I] can be used as a photoredox catalyst to produce phenol from benzene in high yield.

Compound [I] can be used as a photoredox catalyst to produce oxidized metabolites of pharmaceutical products.

When compound [I] is used as a photoredox catalyst, compound [I] can be added in an amount of 0.001 mol to 10 mol equivalent per mol of the substrate.

### Method for producing phenols

The method for producing phenols using compound [I] as a photoredox catalyst is a method for producing phenols, comprising an oxidation step of converting aromatic compounds to phenols by oxidation, characterized in that in the oxidation step, aromatic compounds are oxidized using the photoredox catalyst of the present invention.

In the production method of the present invention, the aromatic compound that serves as a raw material for phenols may have substituents. When the aromatic compound has substituents, the number of substituents is not limited as long as there is one or more points of conversion to phenols. Specifically, the aromatic compound may have one substituent, or two or more substituents. When the aromatic compound has two or more substituents, the substituents may be the same or different. Examples of such substituents include halogen, alkyl, alkoxy, carboxy, and the like. The aromatic ring serving as the skeleton of the aromatic compound is not particularly limited, but examples include benzene, naphthalene, anthracene, phenanthrene, pyrene, and fullerene. Specific examples of the aromatic compound include benzene, fluorobenzene, chlorobenzene, bromobenzene, toluene, o-xylene, m-xylene, p-xylene, mesitylene, ethylbenzene, naphthalene, 1-chloronaphthalene, 2-chloronaphthalene, 1-bromonaphthalene, 2-bromonaphthalene, 1-methylnaphthalene, 2-methylnaphthalene, anthracene, phenanthrene, pyrene, and the like.

In the oxidation step, the photoredox catalyst of the present invention oxidizes aromatic compounds and converts them into phenols, as described above. For example, the oxidation reaction proceeds by photoexcitation of the photoredox catalyst of the present invention. The irradiation light in the photoreaction is also not particularly limited, but is preferably visible light in terms of further simplicity of the reaction etc. More specifically, it is more preferable that the photoredox catalyst of the present invention has an absorption band in the visible light region and can be excited by visible light. Of the wavelengths of the visible light to be irradiated, a more preferred wavelength depends on the absorption band of the photoredox catalyst of the present invention; however, it is more preferably, for example, 300 to 450 nm, even more preferably 360 to 450 nm, and particularly preferably 365 to 435 nm.

The reaction temperature in the oxidation step is also not particularly limited, but is, for example, -100 to 250°C, preferably 0 to 40°C, and more preferably 0 to 30°C. For example, the oxidation reaction can be accelerated by irradiation with visible light at room temperature.

The photoreaction can be easily carried out by using, for example, visible light contained in natural light, such as sunlight. Instead of or in addition to the natural light, for example, a light source, such as an LED light, a xenon lamp, a halogen lamp, a fluorescent lamp, or a mercury lamp, may be used as appropriate. Furthermore, a filter that cuts off wavelengths other than the necessary wavelengths may be used as appropriate.

The disclosures of all patent and non-patent literature cited herein are incorporated herein by reference in their entirety.

### Examples

The present invention is described in more detail with reference to the Test Examples, Reference Examples, and Examples shown below. However, these examples do not limit the present invention and may be changed without deviating from the scope of the present invention.

The following abbreviations may be used in the present specification.

**Table 1**

| Abbreviation | Phrase |
|---|---|
| REX | Reference Example number |
| EX | Example number |
| STR | Structural formula |
| RProp | Production method (the number indicates that the compound was produced using corresponding raw materials, as in the Reference Example compound having that number as the Reference Example number) |
| Prop | Production method (the number indicates that the compound was produced using corresponding raw materials, as in the Example compound having that number as the Example number) |
| Data | Physical property data (NMR1: δ (ppm) in ¹H-NMR in DMSO-d₆; NMR2: δ (ppm) in ¹H-NMR in CDCl₃; NMR3: δ (ppm) in ¹H-NMR in CD₃CN) |
| AcOEt | Ethyl acetate |
| AlCl₃ | Aluminum chloride |
| BrettPhos Pd G3 | [(2-Di-cyclohexylphosphino-3,6-dimethoxy-2',4',6'-triisopropyl-1,1-biphenyl)-2-(2'-amino-1,1'-biphenyl)]palladium(II) methanesulfonate |
| DCM | Dichloromethane |
| Hexane | Hexane |
| IPE | Isopropyl ether |
| CH₃I | Methyl iodide |
| NaH | Sodium hydride |
| NaClO₄ | Sodium perchlorate |

The term "room temperature" in the following Examples generally refers to about 10°C to about 35°C. Ratios shown for mixed solvents indicate volume ratios unless otherwise specified. % indicates wt% unless otherwise specified.

¹HNMR (proton nuclear magnetic resonance) spectra were measured by Fourier transform NMR (Bruker AVANCE III 400 (400 MHz) or Bruker AVANCE III HD (500 MHz)).

### Reference Examples

### Reference Example 1: Production of 4-fluoro-N-(4-fluorophenyl)-N-methylaniline

4-Fluoro-N-(4-fluorophenyl)benzenamine (3.80 g) was dissolved in DMF (30 mL), and NaH (2.424 g) was added under ice-cooling, followed by stirring at room temperature for 15 minutes. After ice-cooling again, CH₃I (1.389 mL) was added, followed by stirring under ice-cooling for 5 minutes, further followed by stirring at room temperature for 1 hour. Water was added to the reaction liquid under ice-cooling, followed by extraction with AcOEt. The organic layer was concentrated, and the residue was purified in a medium-pressure column (Biotage Sfar D 50g, Hexane/AcOEt), thereby obtaining a target product (3.77 g).

Using corresponding raw material compounds, the compound of Reference Example 2 was produced in the same manner as in Reference Example 1.

### Reference Example 3: Production of 4-fluoro-N,N-bis(4-fluorophenyl)-2-methylaniline

Bis(4-fluorophenyl)amine (1.0 g) and 2-bromo-5-fluorotoluene (0.724 mL) were dissolved in toluene (5 mL), and BrettPhos Pd G3 (0.022 g) and sodium tert-butoxide (1.171 g) were added, followed by stirring in a nitrogen atmosphere with heating under reflux. The disappearance of the raw materials was confirmed by LC-MS. Water was added to the reaction liquid. After stirring for a while, the resultant was filtered through Celite, and the filtrate was extracted with AcOEt. The organic layer was concentrated, and the residue was purified in a medium-pressure column (Yamazen Hi-Flash Column, 2L, Hexane/AcOEt), thereby obtaining a target product (1.21 g).

Table 2 shows the structural formulas and physicochemical data of the compounds of Reference Examples 1 to 3.

**Table 2**

| REx | Str | RProp | Data |
|---|---|---|---|
| 1 | 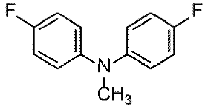 | 1 | NMR2: 6.99-6.94 (m, 4H), 6.93-6.89 (m, 4H), 3.23(s, 3H) |
| 2 | 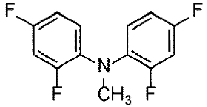 | 1 | NMR2: 7.00-6.94(m, 2H), 6.83-6.77(m, 4H), 3.22(s, 3H) |
| 3 | 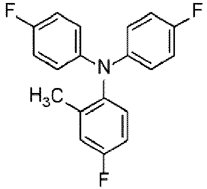 | 3 | NMR2: 7.03(dd, J=8.68, 5.44 Hz, 1H), 6.96-6.84(m, 10H) |

### Examples

### Example 1 (1): Production (1) of 2,7-difluoro-10-methyl-9-(perfluorophenyl)acridin-10-ium perchlorate

AlCl₃ (0.608 g) was added to a DCM (20 mL) solution of 4-fluoro-N-(4-fluorophenyl)-N-methylaniline (1.0 g), followed by stirring in a nitrogen atmosphere at room temperature for 15 minutes. The reaction liquid was ice-cooled, a DCM (3.0 mL) solution of pentafluorobenzoyl chloride (0.945 mL) was added dropwise with a dropping funnel over 15 minutes, and the mixture was heated to room temperature. After 22 hours, water was added to the reaction liquid, followed by washing with hexane. A 1.0 M NaClO₄ aqueous solution was added to the aqueous layer, followed by extraction with DCM. The organic layer was separated and concentrated. The residue was dispersed and washed with IPE, thereby obtaining a target product (0.212 g).

### Example 1 (2): Production (2) of 2,7-difluoro-10-methyl-9-(perfluorophenyl)acridin-10-ium perchlorate

AlCl₃ (547 mg) was added to a mixture of 4-fluoro-N-(4-fluorophenyl)-N-methylaniline (1.00 g) and pentafluorobenzoyl chloride (945 pL), followed by stirring at room temperature. A 1.0 M NaClO₄ aqueous solution was added to the reaction liquid, followed by washing with hexane. The target product was extracted with DCM from the aqueous layer. The organic layer was separated and concentrated. The residue was crystallized with DCM/IPE, followed by filtration, thereby obtaining a target product (0.070 g).

### Example 5. Production of 2,7-difluoro-10-methyl-9-phenylacridin-10-ium perchlorate

In a MW test tube, 4-fluoro-N-(4-fluorophenyl)-N-methylaniline (300 mg) was dissolved in chlorobenzene (5 mL), and benzoyl chloride (189 µL) and Tf-OH (trifluoromethanesulfonic acid) (122 µL) were added, followed by stirring under microwave irradiation at 160°C for 1 hour. The reaction liquid was diluted with DCM, and the organic layer was washed with water and a 1 M NaClO₄ aqueous solution, and concentrated. The residue was dissolved in a small amount of DCM, and IPE was added to precipitate crystals. Then, IPE was further added, and the crystals were filtered and washed with IPE, thereby obtaining a target product (140 mg).

### Example 20. Production of 10-(4-fluorophenyl)-9-phenylacridin-10-ium perchlorate

10-(4-fluorophenyl)acridin-9(10H)-one (300 mg) was dissolved in THE (10 mL), and phenylmagnesium bromide (691 µL) was added under ice-cooling in a nitrogen atmosphere, followed by stirring at room temperature. The disappearance of the raw materials was confirmed, and the reaction liquid was concentrated. The residue was dissolved in DCM, washed with water and 1 M NaClO₄, and concentrated. The residue was dissolved in a small amount of DCM, and IPE was gradually added to precipitate crystals. The crystals were filtered and washed with IPE, thereby obtaining a target product (174 mg).

Using corresponding raw material compounds, the compounds of Example 2 to 4, 6 to 19, and 21 were produced in the same manner as in Examples 1, 5, and 20.

Tables 3 to 8 show the structural formula and physicochemical data of the compounds of Examples 1 to 21. In the tables, "Prop 1 (1)" refers to Example 1 (1), and "Prop 1 (2)" refers to Example 1 (2).

**Table 3**

| Ex | Str | Prop | Data |
|---|---|---|---|
| 1 | 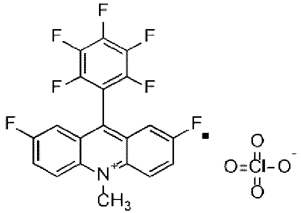 | 1 (1) | NMR1; 9.09 (dd, J = 10. 1, 4.40 Hz, 2H), 8.55-8.51(m, 2H), 8.16(br d, J = 7.25 Hz, 2H), 5.04 (s, 3H) |
| 2 | 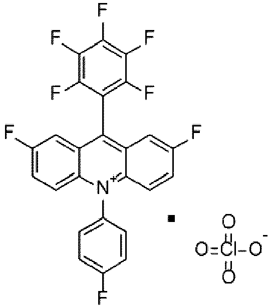 | 1 (1) | NMR2; 7.97-7.94(m, 4H), 7.74(dd, J = 10.1, 4.43 Hz, 2H), 7.63-7.56(m, 4H) |
| 3 | 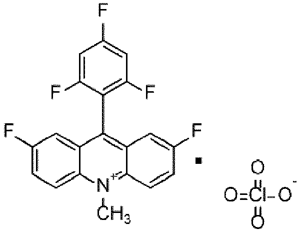 | 1 (2) | NMR3; 8.78 (dd, J = 10.2, 4.42 Hz, 2H), 8.32-8.27 (m, 2H), 7.80-7.77 (m, 2H), 7.35-7.29 (m, 2H), 4.92 (s, 3H) |

**Table 4**

| | | | |
|---|---|---|---|
| 4 | 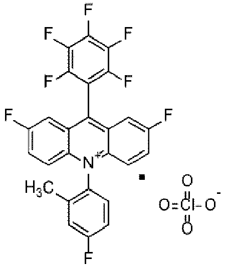 | 1 (2) | NMR2: 8.16(dd, J = 8. 88, 5.02 Hz, 1H), 8.02-7.97(m, 2H), 7.71(dd, J = 10.0, 4.41 Hz, 2H), 7.60(br d, J = 7.26 Hz, 2H), 7.47(td, J = 8.06. 3.10 Hz, 1H), 7.40(dd, J = 8.37, 2.32 Hz, 1H), 1.83(s, 3H) |
| 5 | 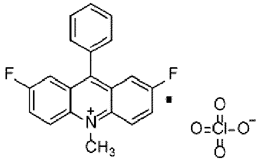 | 5 | NMR2; 8.83 (dd, J=10.1, 4.31 Hz, 2H), 8.15-8.12 (m, 2H), 7.76-7.12 (m, 3H), 7.54 (dd, J = 8.39, 2.72 Hz, 2H), 7.49 (br d, J = 8.39 Hz, 2H), 5.10 (s, 3H) |
| 6 | 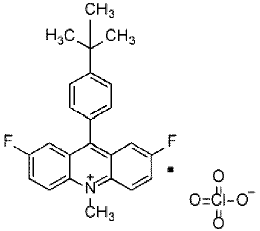 | 5 | NMR2; 8.80 (dd, J = 9.97, 4.09 Hz, 2H), 8.16-8.12 (m, 2H), 7.74 (d, J = 7.97 Hz, 2H), 7.63 (dd, J = 8.52, 2.47 Hz, 2H), 7.41 (d, J = 7.97 Hz, 2H), 5.10 (s, 3H), 1.49 (s, 9H) |
| 7 | 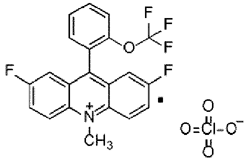 | 5 | NMR2; 8.84 (dd, J = 9.95, 4.15 Hz, 2H), 8.16-8.12 (m, 2H), 7.68-7.65 (m, 2H), 7.56 (br d, J = 7.05 Hz, 1H), 7.39 (dd, J = 8.14, 2.49 Hz, 2H), 5.12 (s, 3H) |

**Table 5**

| | | | |
|---|---|---|---|
| 8 | 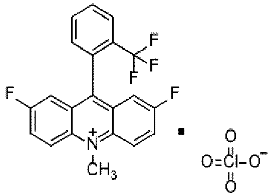 | 5 | NMR2; 8.75 (dd, J = 9.95, 4.05 Hz, 2H), 8.14-8.10 (m, 2H), 8.07-8.06 (m, 2H), 7.92-7.90 (m, 2H), 7.59-7.57 (m, 2H), 7.23 (dd, J = 8.11, 2.46 Hz, 2H), 5.10 (s, 3H) |
| 9 | 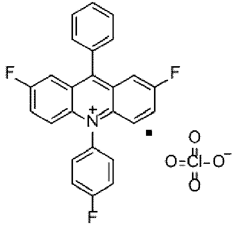 | 5 | NMR2; 7.88-7.81 (m, 4H), 7.75-7.74 (m, 3H), 7.70-7.68 (m, 2H), 7.64 (dd, J = 8.51, 2.38 Hz, 2H), 7.59-7.55 (m, 4H) |
| 10 | 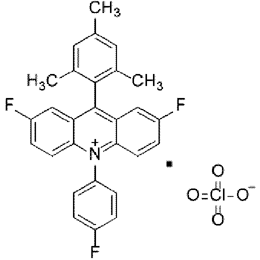 | 5 | NMR2; 7.97 (dd, J = 8.47, 4.42 Hz, 2H), 7.89-7.85( m, 2H), 7.67 (dd, J = 9.84, 4.17 Hz, 2H), 7.60 (t, J = 8.24 Hz, 2H), 7.47 (dd, J = 8.04, 2.34 Hz, 2H), 7.19 (s, 2H), 2.50 (s, 3H), 1.90 (s, 6H) |
| 11 | 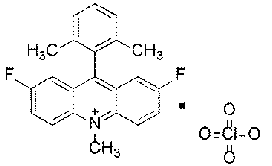 | 5 | NMR2; 8.95 (dd, J = 9.93, 4.10 Hz, 2H), 8.19-8.15 (m, 2H), 7.53 (t, J = 8.70 Hz, 1H), 7.37-7.33 (m, 4H), 5.19 (s, 3H), 1.80 (s, 6H) |

**Table 6**

| | | | |
|---|---|---|---|
| 12 | 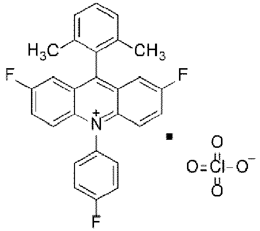 | 5 | NMR2; 7.95 (dd, J = 8.63, 4.35 Hz, 2H), 7.89-7.85 (m, 2H), 7.68 (dd, J = 8.90, 4.35 Hz, 2H), 7.59-7.53 (m, 3H), 7.42 (dd, J = 8.08, 2.47 Hz, 2H), 7.38 (d, J = 7.70 Hz, 2H), 1.93 (s, 6H) |
| 13 | 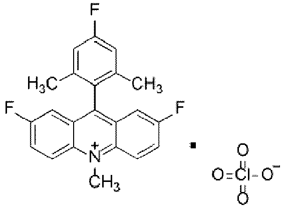 | 5 | NMR2; 8.99 (dd, J = 9.96, 4.09 Hz, 2H), 8.18-8.14 (m, 2H), 7.34 (dd, J = 7.96, 2.49 Hz, 2H), 7.11 (d, J = 9.06 Hz, 2H), 5.17 (s, 3H), 1.80 (s, 6H) |
| 14 | 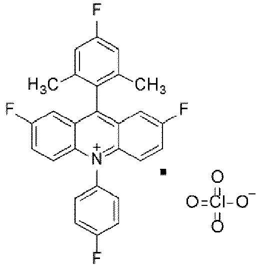 | 5 | NMR2; 7.95 (dd, J = 8.70, 4.40 Hz, 2H), 7.90-7.86 (m, 2H), 7.68 (dd, J = 9.91, 4.28 Hz, 2H), 7.58 (t, J = 8.26 Hz, 2H), 7.41 (dd, J = 8.07, 2.53 Hz, 2H), 7.12 (d, J = 9.04 Hz, 2H), 1.94 (s, 6H) |
| 15 | 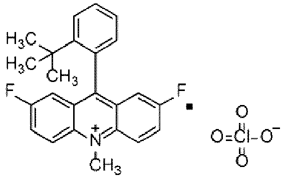 | 5 | NMR2; 8.83 (dd, J = 9.98, 4.17 Hz, 2H), 8.15-8.11 (m, 2H), 7.86 (d, J = 8.40 Hz, 1H), 7.68 (t, J = 7.44 Hz, 1H), 7.43-7.39 (m, 3H), 6.98 (d, J = 7.68 Hz, 2H), 5.14 (s, 3H), 2.49 (s, 3H), 1.00 (s, 9H) |

**Table 7**

| | | | |
|---|---|---|---|
| 16 | 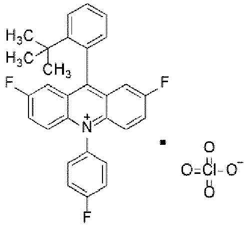 | 5 | NMR2; 8.24-8.21 (m, 1H), 7.85-7.81 (m, 3H), 7.67 (t, J = 7.42 Hz, 1H), 7.62-7.58(m, 4H), 7.54 (td, J = 8.36, 2.49 Hz, 1H), 7.49-7.46 (m, 3H), 7.41 (d, J = 7.47 Hz, 1H), 5.14 (s, 3H), 1.06 (s, 9H) |
| 17 | 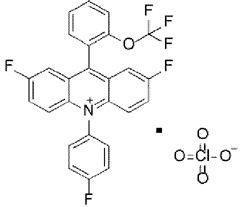 | 5 | NMR2; 8.28-8.25 (m, 1H), 7.98 (d, J = 7.65 Hz, 1H), 7.88-7.82 (m, 3H), 7.72 (t, J = 7.66 Hz, 1H), 7.66 (d, J = 8.46 Hz, 1H), 7.63-7.60 (m, 3H), 1.57-7.56 (m, 2H), 7.49 (dd, J = 8.30, 2.39 Hz, 2H) |
| 18 | 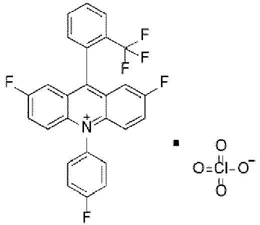 | 5 | NMR2; 8.32-8.29 (m, 1H), 8.06 (d, J = 7.84 Hz, 1H), 8.02 (d, J = 7.42 Hz, 1H), 7.96 (t, J = 7.44 Hz. 1H), 7.91 (t, J = 7.68 Hz, 1H), 7.86-7.82 (m, 2H), 7.64-7.59 (m, 3H), 7.54 (d, J = 5.61 Hz, 2H), 7.33 (dd, J = 8.12, 2.32 Hz, 2H) |
| 19 | 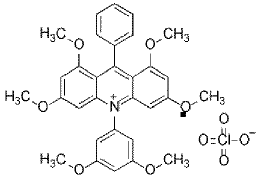 | 5 | NMR2; 7.44-7.38 (m, 3H), 7.26 (dt, J = 6.71, 1.44 Hz, 2H), 6.82 (t, J = 2.18 Hz, 1H), 6.69 (d, J = 2.18 Hz, 2H), 6.43 (d, J = 2.30 Hz, 2H), 6.18 (d, J = 2.30 Hz, 2H), 3.92 (s, 6H), 3.82 (s, 6H), 3.41 (s, 6H) |

**Table 8**

| | | | |
|---|---|---|---|
| 20 | 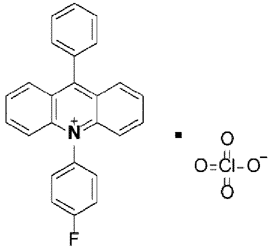 | 20 | NMR2; 8.01-8.05 (m, 4H), 7.84-7.81 (m, 2H), 7.77-7.71 (m, 7H), 7.58-7.52 (m, 4H) |
| 21 | 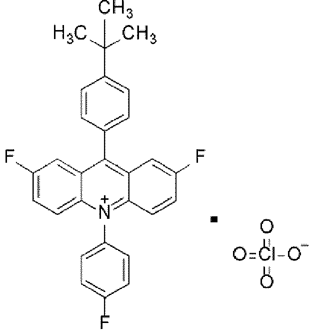 | 1 (1) | NMR2; 7.85-7.79 (m, 4H), 7.76-7.68 (m, 4H), 7.62-7.60 (m, 2H), 7.58-7.53 (m, 4H), 1.49 (s, 9H) |

Using corresponding raw material compounds, the compounds of Examples 22 to 40 can be produced in the same manner as in Example 1, 5, or 20. Tables 9 to 12 show the structural formulas of the compounds of Examples 22 to 40.

**Table 9**

| Ex | Str | Ex | Str |
|---|---|---|---|
| 22 | 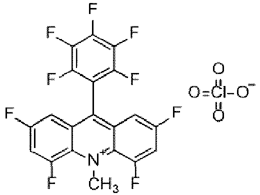 Election Affinity (eV): -6.44 | 23 | 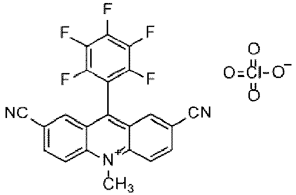 Election Affinity (eV): -6.55 |
| 24 | 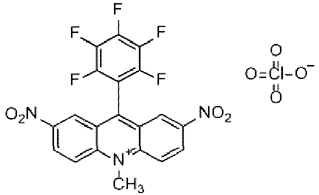 Election Affinity (eV): -6.88 | 25 | 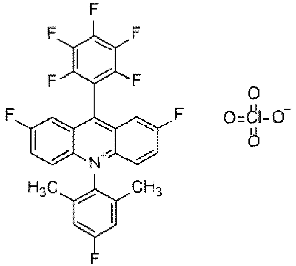 Election Affinity (eV): -5.99 |
| 26 | 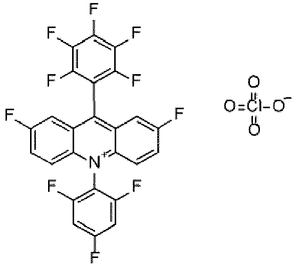 Election Affinity (eV): -6.12 | 27 | 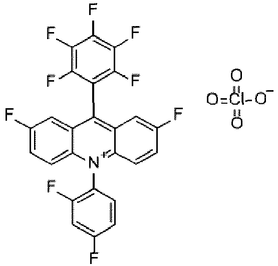 Election Affinity (eV): -6.06 |

**Table 10**

| | | | |
|---|---|---|---|
| 28 | 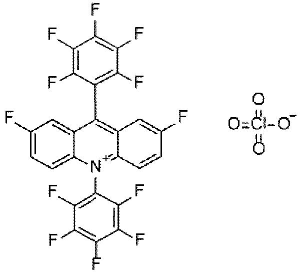 Election Affinity (eV): -6.30 | 29 | 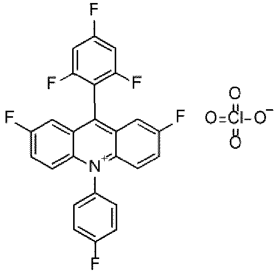 Election Affinity (eV): -5.80 |
| 30 | 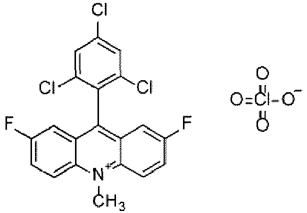 Election Affinity (eV): -5.83 | 31 | 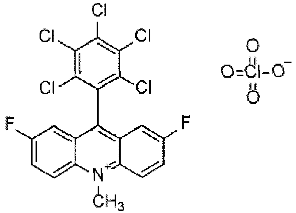 Election Affinity (eV): -5.92 |
| 32 | 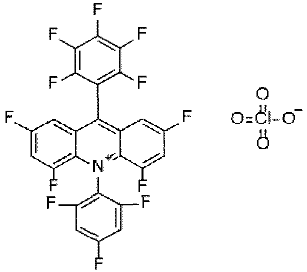 Election Affinity (eV): -6.43 | 33 | 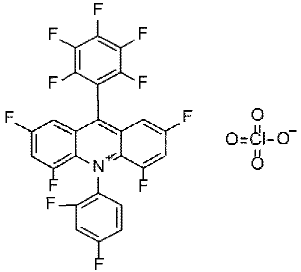 Election Affinity (eV): -6.46 |

**Table 11**

| | | | |
|---|---|---|---|
| 34 | 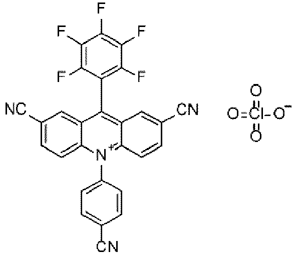 | 35 | 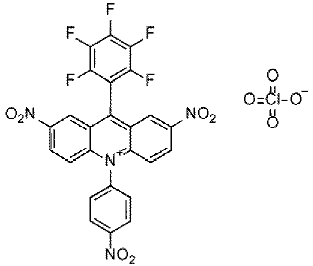 |
| | Election Affinity (eV): -6.63 | | Election Affinity (eV): -6.79 |
| 36 | 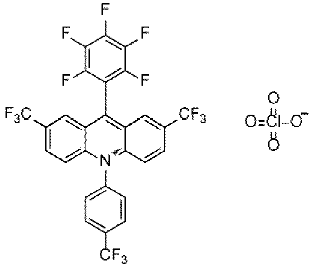 | 37 | 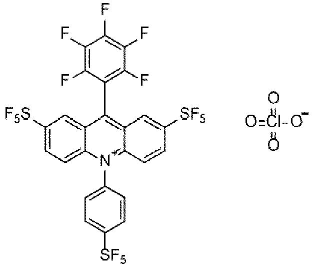 |
| | Election Affinity (eV): -6.34 | | Election Affinity (eV): -6.61 |
| 38 | 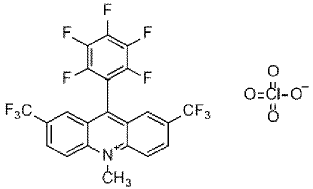 | 39 | 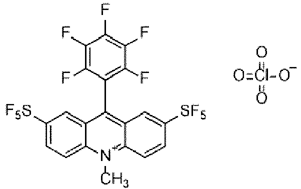 |
| | Election Affinity (eV): -6.31 | | Election Affinity (eV): -6.54 |

**Table 12**

| | | | |
|---|---|---|---|
| 40 | 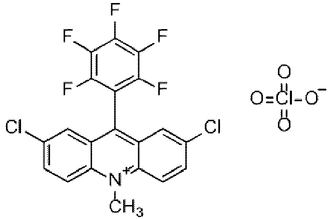 | 41 | 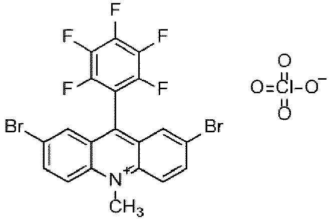 |
| | Election Affinity (eV): -6.07 | | Election Affinity (eV): -6.06sh |

### Test Examples

The test results of representative compounds of the present invention are shown below, and the effects of the compounds are explained; however, the present invention is not limited to these Test Examples.

### Test Example 1 (Measurement of reduction potential at the singlet excited state)

### - Sample preparation

The compounds of the present invention or the comparative compounds shown in Table 13 below (5 mmol/L) and TBAPF6 (100 mmol/L) were dissolved in acetonitrile (1 mL) to prepare samples.

### - Cyclic voltammetry (CV) measurement

Each sample was injected into a volumetric cell, electrodes were attached in an argon atmosphere, and the cell was set in a device and measured. The one-electron reduction potential (E_{red}) was determined from the average of the peak potentials.
Electrolyte: tetrabutylammonium hexafluorophosphate (TBAPF6)
Working electrode: platinum disk electrode (produced by BAS)
Counter electrode: platinum wire (produced by BAS)
Reference electrode: Ag/AgNO₃ (produced by BAS)
Sweep speed: 100 mV/s
Device: Electrochemical Analyzer Model 610E (produced by BAS)

### - Maximum absorption wavelength measurement

Each sample was measured using a UV-visible spectroscopy system 8454 (produced by Agilent Technologies).

### - Maximum fluorescence wavelength measurement

Each sample was measured using a FluoroMax-4P fluorescence spectrometer (produced by HORIBA, Ltd.). - Reduction potential at the singlet excited state was calculated.

Singlet excitation energy was calculated from the maximum absorption wavelength and maximum fluorescence wavelength. The singlet excitation energy was added to the one-electron reduction potential determined by cyclic voltammetry to calculate the reduction potential at the singlet excited state.

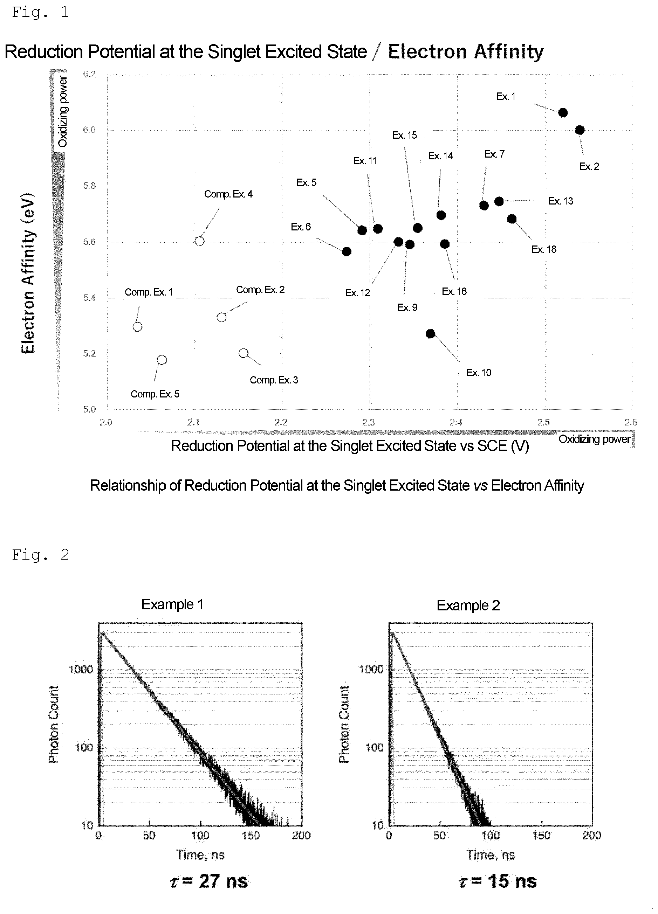

Fig. 1 shows the results.

**Table 13**

| | | | |
|---|---|---|---|
| Comparative Example 1 | 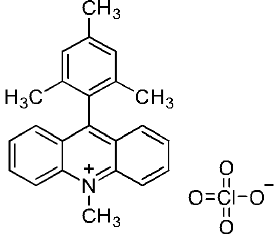 | Comparative Example 2 | 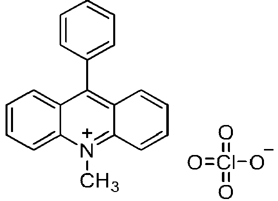 |
| Comparative Example 3 | 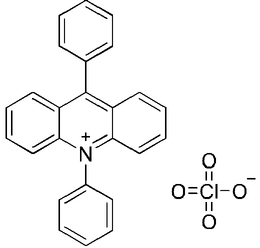 | Comparative Example 4 | 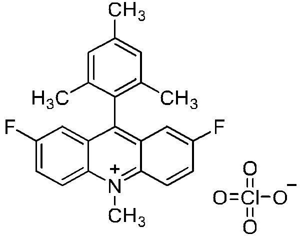 |
| Comparative Example 5 | 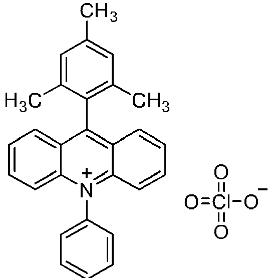 | | |

### Test Example 2 (Measurement of fluorescence lifetime)

The compounds of the present invention (0.1 mmol/L) were each dissolved in acetonitrile (3 mL), sealed in a 1-cm square cell, and replaced in an argon atmosphere to prepare samples. The samples were measured using a fluorescence lifetime system (DeltaFlex: produced by HORIBA, Ltd.).

Fig. 2 shows the measurement results.

### Test Example 3 (DFT calculation: electron affinity)

DFT calculations were performed using the following system.
Hardware: High-Performance Computer (produced by HPC Systems, Inc.)
Software: Gaussian 09 Rev D.01
The following calculations were performed.
1. Structural optimization (cation) (M06-2X/6-31++G(d)) and energy value
2. HOMO and LUMO levels
3. Structural optimization (neutral radical) (UM06-2X/6-31++G(d)) and energy value
4. Calculation of electron affinity

Fig. 1 shows the results.

### Test Example 4 (Production of phenol)

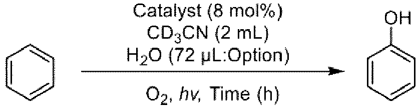

The compound of the present invention (8 mol%), benzene (0.1 M), water (72 µL, optional), and a deuterated acetonitrile solution (2 mL) were irradiated with an LED lamp (product name: Aldrich (trademark) Micro Photochemical Reactor, produced by Merck) in an oxygen atmosphere using the wavelength and irradiation time shown in Table 14. Further, Acr⁺-Mes (Comparative Example 1) was used for comparison.

Table 14 shows the results. The yield indicates the yield of phenol produced, and the residual ratio indicates the ratio of benzene used as a raw material.

The yield and residual ratio were calculated from the NMR chart using 1,3,5-trimethoxybenzene as the internal standard reagent.

**Table 14**

| Example No. | Irradiation wavelength (nm) | Time (h) | Water addition* | Yield (%) | Residual ratio (%) |
|---|---|---|---|---|---|
| 1 | 405 | 66 | Not added | 32 | 2 |
| 1 | 435 | 66 | Not added | 24 | 0 |
| 1 | 365 | 15 | Not added | 38 | 4 |
| 1 | 405 | 15 | Not added | 70 | 15 |
| 1 | 435 | 15 | Not added | 73 | 12 |
| 1 | 435 | 15 | Added | 80 | 12 |
| 2 | 435 | 15 | Added | 63 | 26 |
| Comparative Example 1 | 365 | 15 | Not added | 0 | 100 |
| Comparative Example 1 | 405 | 15 | Not added | 0 | 100 |

| | | | | | |
|---|---|---|---|---|---|
| * Water was added at the beginning of the reaction and 10 hours after the start of the reaction. | | | | | |

## Claims

1. A compound represented by formula [I]: 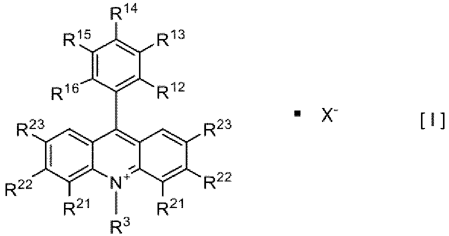 wherein
R¹², R¹³, R¹⁴, R¹⁵, and R¹⁶ are the same or different and are each hydrogen, halogen, C₁₋₆ alkyl optionally substituted with halogen, or C₁₋₆ alkoxy optionally substituted with halogen;
R²¹, R²², and R²³ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano;
R³ is C₁₋₆ alkyl or 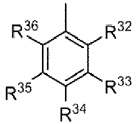 wherein R³², R³³, R³⁴, R³⁵, and R³⁶ are the same or different and are each hydrogen, halogen, C₁₋₆ alkyl optionally substituted with halogen, C₁₋₆ alkoxy optionally substituted with halogen, sulfanyl optionally substituted with halogen, nitro, or cyano; and
X⁻ is an anion;
provided that the following cases are excluded:
1) all of R¹², R¹³, R¹⁴, R¹⁵, R¹⁶, R²¹, R²², and R²³ are hydrogen, and R³ is methyl or phenyl;
2) all of R¹², R¹⁴, R¹⁶, and R³ are methyl, and R²³ is hydrogen or fluorine; and
3) R¹² is hydrogen or methyl, R¹³ is hydrogen or methyl, R¹⁴ is hydrogen or methyl, R¹⁵ is hydrogen or methyl, R¹⁶ is hydrogen or methyl, R³ is methyl or unsubstituted phenyl, and R²³ is hydrogen.

2. The compound according to claim 1, wherein
R¹², R¹³, R¹⁴, R¹⁵, and R¹⁶ are the same or different and are each hydrogen, fluorine, chlorine, methyl, t-butyl, trifluoromethyl, or trifluoromethoxy;
R²¹, R²², and R²³ are the same or different and are each hydrogen, fluorine, chlorine, bromine, trifluoromethyl, methoxy, pentafluorosulfanyl, nitro, or cyano; and
R³ is methyl or 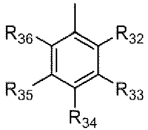 wherein R³², R³³, R³⁴, R³⁵, and R³⁶ are the same or different and are each hydrogen, fluorine, chlorine, bromine, methyl, trifluoromethyl, methoxy, pentafluorosulfanyl, nitro, or cyano.

3. The compound according to claim 1, wherein
R¹², R¹³, R¹⁴, R¹⁵, and R¹⁶ are the same or different and are each hydrogen, fluorine, methyl, t-butyl, trifluoromethyl, or trifluoromethoxy;
R²¹ and R²² are hydrogen, R²³ is fluorine; and
R³ is methyl or 4-fluorophenyl.

4. The compound according to claim 1 selected from the following:

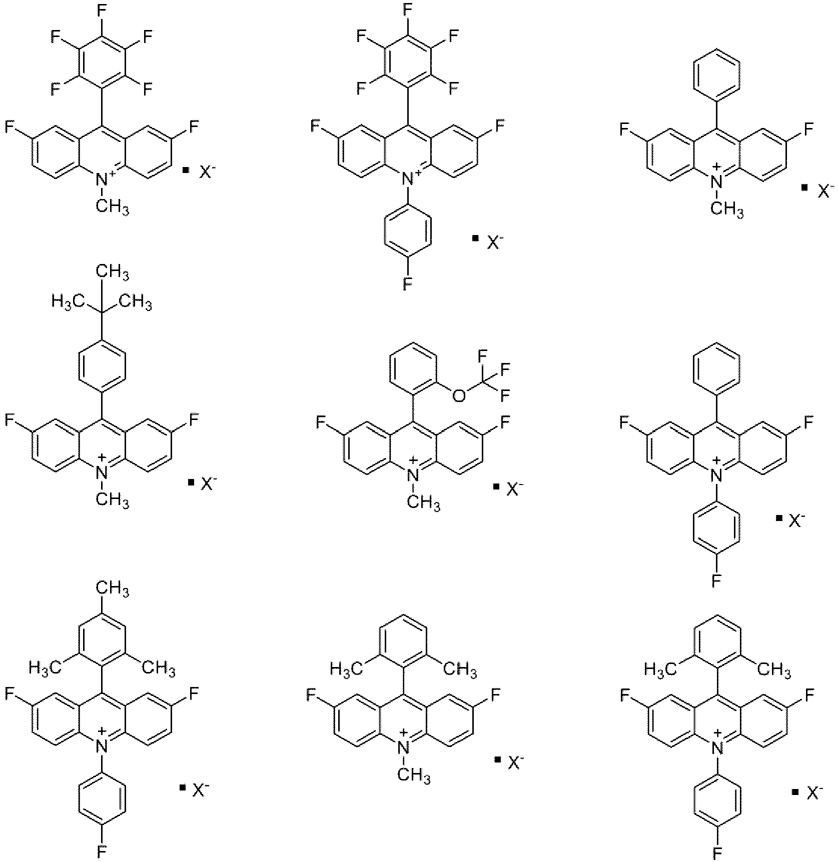

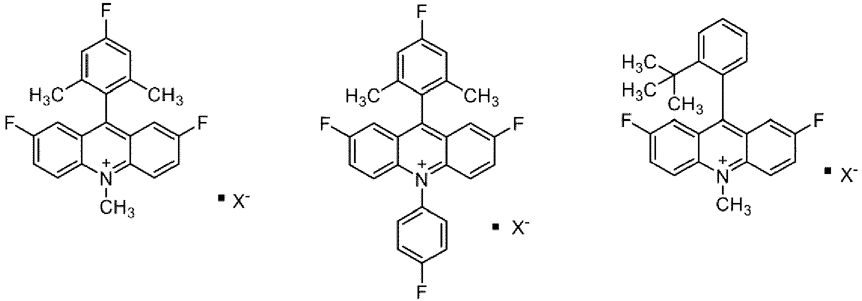

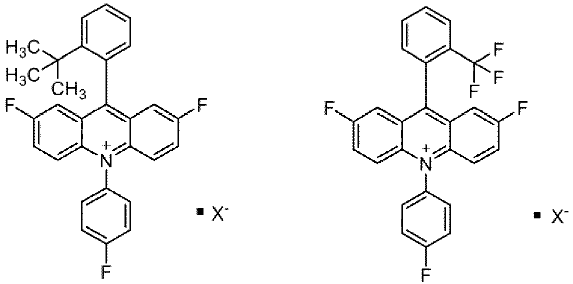

5. The compound according to claim 1, wherein X⁻ is perchlorate ion (ClO₄⁻), hexafluorophosphate ion (PF₆⁻), or tetrafluoroborate ion (BF₄⁻).

6. A photoredox catalyst selected from the compound according to any one of claims 1 to 5.

7. Use of the compound according to any one of claims 1 to 5 as a photoredox catalyst.

8. A method for producing phenol from optionally substituted benzene, comprising irradiating optionally substituted benzene with visible light in the presence of the compound according to any one of claims 1 to 5.
